# EUROPEAN PATENT APPLICATION

(11) **EP 1 195 434 A1**
(43) Date of publication of application: **10.04.2002**
(21) Application number: 00944309.4
(22) Date of filing: 06.07.2000
(51) Int. Cl.: C12N 15/09

(54) **METHOD FOR CONSTRUCTING FULL-LENGTH cDNA LIBRARIES**

(30) Priority: 08.07.1999 JP 19510499
(71) Applicant: Helix Research Institute, Kisarazu-shi, Chiba 292-0812 (JP)
(72) Inventor: OTA, Toshio, Kyowa Hakko Kogyo Co.,Ltd., Machida-shi Tokyo 194-8533 (JP); WAKAMATSU, Ai, Kisarazu-shi, Chiba 292-0014 (JP); ISOGAI, Takao, Inashiki-gun, Ibaraki 300-0303 (JP); OTSUKA, Kaoru, Honjou-shi, Saitama 367-0047 (JP); SUZUKI, Yutaka, Tokyo 108-0072 (JP); SUGANO, Sumio, Tokyo 167-0052 (JP)
(74) Representative: VOSSIUS & PARTNER
(86) International application number: JP0004517
(87) International publication number: WO0104286

(57) **Abstract**

The present inventors have developed methods that enable the construction of cDNA libraries with extremely high fullness ratio from a very small amount of starting as compared with conventional methods by optimizing reaction conditions in the steps of the alkaline phosphatase reaction, acid pyrophosphatase reaction, and RNA ligase reaction of conventional oligo-capping methods.

SEQUENCE LISTING

<110> Helix Research Institute

<120> Method for constructing full-length cDNA libraries

<130> H1-102PCT

<140>

<141>

<150> JP 1999-195104

<151> 1999-07-08

<160> 6

<170> PatentIn Ver. 2.0

<210> 1

<211> 30

<212> RNA

<213> Artificial Sequence

<220>

<223> Description of Artificial Sequence:Artificially
Synthesized RNA Sequence

<400> 1

agcaucgagu cggccuuguu ggccuacugg                                        30

<210> 2

<211> 42

<212> DNA

<213> Artificial Sequence

<220>

<223> Description of Artificial Sequence:Artificially

      Synthesized Primer Sequence

<400> 2

gcggctgaag acggcctatg tggccttttt tttttttttt tt                          42

<210> 3

<211> 21

<212> DNA

<213> Artificial Sequence

<220>

<223> Description of Artificial Sequence:Artificially

      Synthesized Primer Sequence

<400> 3

agcatcgagt cggccttgtt g                                                 21

<210> 4

<211> 21

<212> DNA

<213> Artificial Sequence

<220>

<223> Description of Artificial Sequence:Artificially

Synthesized Primer Sequence

<400> 4

tgctactgtg tcggggttgt a 21

<210> 5

<211> 21

<212> DNA

<213> Artificial Sequence

<220>

<223> Description of Artificial Sequence:Artificially

Synthesized Primer Sequence

<400> 5

cctgaaccat ccaggccaaa t 21

<210> 6

<211> 21

<212> DNA

<213> Artificial Sequence

<220>

<223> Description of Artificial Sequence:Artificially

Synthesized Primer Sequence

<400> 6

gcggctgaag acggcctatg t 21

## Description

### Technical Field

The present invention relates to methods for constructing cDNA libraries.

### Background Art

Full-length cDNA clones are important tools for elucidating the primary structures of transcripts and translation products of genes, and for corroboratively analyzing gene functions. More specifically, the protein structure encoded by a full-length cDNA can be easily predicted by analyzing the full-length cDNA, since it contains all the information about the primary structure of its translation product, namely the protein. Furthermore, full-length cDNA can be used to produce the protein encoded by the cDNA in large quantities, by using an appropriate expression system, or to analyze its function as biological activities, by introducing it into appropriate cell lines.

Full-length cDNA libraries are useful for efficiently obtaining the full-length cDNA. However, it is very difficult to efficiently obtain full-length cDNA from cDNA libraries that were constructed by conventional methods. Among the methods for constructing cDNA libraries, the method of Gubler and Hoffman (Gubler, U. and Hoffman, B. J. (1983) "A simple and very efficient method for generating cDNA libraries." Gene 25: 263-269) is the most common method at present, since cDNA clones containing relatively long sequence can be obtained according to this method. In this method, cDNA corresponding to the mRNA at the 5'-end portion cannot in principle be synthesized, because a nicked mRNA annealed to the first strand is used as the primer for the second strand cDNA synthesis. In contrast, cDNA corresponding to the mRNA at the 5'-end portion can be theoretically obtained by the Okayama-Bergmethod (Okayama, H. and Berg, P. (1982) Biotechnology 24:210-219) , wherein the second strand cDNA synthesis is primed from the vector sequence. However, it is difficult to obtain long cDNA sequences according to this method, and it is reported that it has a low cloning efficiency. Moreover, what is more problematic is that mRNA extracted frombiological samples include a high degree of degraded mRNA with incomplete length.

The methods mentioned above allow synthesis of cDNA with incomplete length derived from degraded mRNA molecules together with full-length cDNA. Thus, it is impossible to construct a cDNA library with a high fullness ratio. Additionally, it is substantially impossible to distinguish between mass-produced non-full-length cDNAs as above and cDNA synthesized from full-length mRNA.

Several methods for effectively producing full-length cDNA have been reported so far. To efficiently obtain full-length cDNA, molecules that recognize the CAP structure, a specific structure of the 5'-end in eukaryotic cells, are selected. Sugano et al. have developed the "oligo-capping" method, wherein the CAP structure is specifically recognized and then is replaced with a synthetic oligonucleotide (K. Maruyama and S. Sugano, (1994) Gene 138: 171-174; Y. Suzuki et al. , (1997) Gene 200; and Yutaka Suzuki and Sumio Sugano, (1996) "cDNA Cloning", pp. 46-51, YODOSHA) . Hayashizaki et al. have developed a method for specifically labeling the CAP structure with a biotin molecule (P. Carninci et al. , (1996) Genomics 37: 327-336; P. Carninci et al., (1996) DNA Research 4: 61-66). In addition to these methods, other methods, such as the "combination of oligo-capping and Okayama-Berg methods" (S. Kato et al., (1994) Gene 150: 243-250) , and the "chemical linker-ligation (Genset) method" (Merenkova, N. et al. "Method for the specific coupling of the CAP of the extremity 5' of a fragment mRNA and preparation of complete CDNA."PCT/FR96/00651, 1996) have been developed. In principle, these methods allow for selective collection of cDNA clones derived from full-length mRNA. Thus, construction of cDNA libraries containing full-length cDNA at a high rate is currently possible by using these methods.

However, all these methods require a larger quantity of starting material as compared to conventional methods for preparing cDNA libraries. In the protocols reported so far, at least about 5 to 10 µg of poly (A) RNA is required as the RNA sample for the oligo-capping method (Y. Suzuki et al., (1997) Gene 200: 149-156; and Yutaka Suzuki and Sumio Sugano, (1996) "cDNA Cloning", pp. 46-51, YODOSHA), and at least about 5 µg of poly (A) RNA sample is required for the cap-trapping method (P. Carninci et al., (1996) Genomics 37: 327-336; P. Carninci et al., (1996) DNA Research 4: 61-66). These methods also confront technical difficulties, such as a marked decrease of the fullness ratio of the resultant cDNA, and a reduction in the titer of the clones obtained, when only small quantities of RNA samples are supplied as the starting materials. Accordingly, it is quite difficult to construct cDNA libraries with high fullness ratio by conventional methods under the condition with small quantity of biological materials.

### Disclosure of the Invention

The object of the present invention is to provide methods for constructing cDNA libraries with a high fullness ratio, even when only a small quantity of RNA sample is used as the starting material.

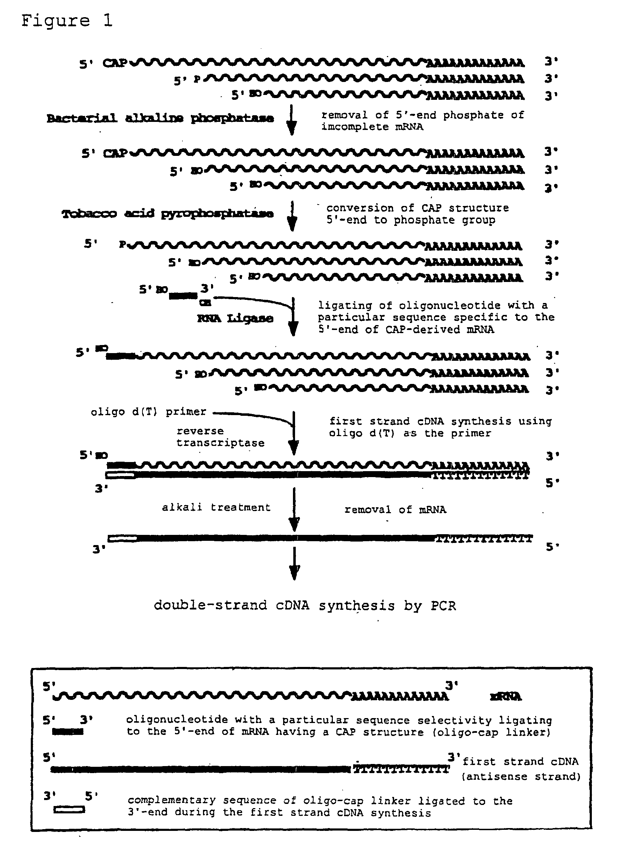

In the conventional oligo-capping method, cDNA libraries containing abundant cDNAs extending from the 5'-end to the 3'-end of the full-length mRNA are constructed using a process comprising the following steps (see Figure 1):
1) first, mRNA samples are treated with bacterial alkaline phosphatase (BAP) to remove the phosphate groups at the 5'-ends of the non-full-length mRNA molecules. According to this treatment, all the 5'-ends of mRNAs (other than those of the full-length mRNA having the 5'-CAP structure) are turned into hydroxyl groups;
2) then, mRNA samples are treated with tobacco acid pyrophosphatase (TAP) to convert the CAP structure at the 5'-end of the full-length mRNA to a phosphate group. According to this treatment, only the full-length mRNAs are made to have a phosphate group at the 5'-end;
3) RNA Ligase is used to ligate synthetic oligo-RNA (oligo-capping linker) to the 5'-end of the full-length mRNAs. This results in addition of synthetic oligo-RNA to the 5'-end of only the full-length mRNAs, which have a phosphate group at their 5'-end; and
4) the first strand cDNAs are synthesized using as templates the mRNA having oligo-capping linker attachments, and then, are subjected to polymerase chain reaction using as primers the sequence in the oligo-capping linker and the oligo-d(T) adapter. As a result, only cDNAs corresponding to the full-length mRNAs are amplified.

The present inventors analyzed plentiful cDNA clones obtained from the constructed oligo-capped library as above, and found that the fullness ratio of a cDNA library constructed in this manner is about 60 to 75% (T. Otaetal., (1998) Microbial and comparative genomics. 3:C-87) . The clone titer and fullness ratio of each cDNA library may largely depend on quantity and quality of the starting material used for construction. When the cDNA libraries are constructed from a small quantity of starting materials, not only the clone titer but also the fullness ratio tends to be markedly reduced.

The present inventors assumed two causes for the reduction in the fullness ratio of the library constructed by the oligo-capping method. Namely, insufficient bacterial alkaline phosphatase reactions dephosphorylating the 5'-ends of non-full-length mRNAs, and degradation of mRNA generating a phosphate group at the 5'-end following the bacterial alkaline phosphatase reaction (step 1 above). Either cause leads to the reduction of the fullness ratio of the cDNA library, resulting from the addition of oligo-capping linkers to the 5'-ends of the non-full-length mRNA molecules degraded irrespective of the presence or absence of the CAP structure to the mRNA in the reaction adding oligo-capping linkers using RNA Ligase.

On the other hand, the clone titer seemed to be reduced by insufficient decapping reaction by tobacco acid pyrophosphatase and by incomplete addition reaction of oligo-capping linkers by RNA Ligase (step 2 above).

Since the percentage of fullness ratio of the cDNA library largely depends on the amount of the starting material, the present inventors considered that reaction conditions for each step of the oligo-capping reaction, in particular, for minimizing degradation of mRNA, should be optimized depending on the state and quantity of the sample. To estimate the accurate fullness ratio of the constructed cDNA library, a measure of clone sequences from the constructed library should be analyzed. However, it is quite inefficient to estimate the fullness ratio of the obtained cDNA library to feed them back to the reaction conditions besides examining reaction conditions for each step of the multi-step reactions for constructing the library.

Therefore, to optimize the reaction conditions for each step of the oligo-capping method and to improve the protocol in an effective manner, the present inventors have developed a system to visualize the products of the oligo-capping reaction with high sensitivity using synthetic oligo-RNA labeled with digoxigenin (DIG). This system allows rapid and sensitive evaluation of the reaction conditions and state of RNA in each step of the oligo-capping method.

The present inventors also improved the protocol to minimize mRNA degradation and optimize reaction conditions for each step, namely the step of alkaline phosphatase reaction, acid pyrophosphatase reaction, and RNA ligase reaction, using this evaluation system to evaluate the state of mRNA, the substrate for oligo-capping reaction, and the progress of the reaction at each step. As a result, the present inventors found that mRNA degradation can be minimized by changing RNA samples in each steps of the above reaction steps from conventionally used poly (A) RNA to total RNA. The present inventors also found that elevating the pH of the reaction mixture for acidpyrophosphate reaction increases the efficiency of mRNA decapping. Based on these findings, the present inventors successfully developed new protocols that enable construction of cDNA libraries with a very high fullness ratio, even if the quantity of the starting material is extremely low compared to those of the conventional methods.

Thus, the present invention relates to oligo-capping methods that allow construction of cDNA libraries with a high fullness ratio, even when a small amount of RNA sample is used as the starting material. More specifically, the present invention provides the following:
(1) a method for constructing a cDNA library, comprising the steps of:
   (a) treating the RNA sample containing mRNA and other RNA with alkaline phosphatase to remove phosphate groups from non-full-length mRNA molecules having phosphate groups at the 5'-ends;
   (b) following the treatment of step (a), treating the RNA sample with acid pyrophosphatase to convert the CAP structures of the full-length mRNAs in the sample into phosphate groups, wherein the full-length mRNAs have a CAP structures at their 5'-ends;
   (c) following the treatment of step (b), treating the RNA sample with RNA Ligase to ligate synthetic oligo-RNA (oligo-capping linkers) to the 5'-ends of mRNAs in the RNA sample, wherein the CAP structures of the mRNAs at the 5'-end are converted into phosphate groups;
   (d) selecting poly (A) RNAs from the RNA sample following the treatment of step (c); and
   (e) performing reverse transcription using the poly (A) RNAs selected in step (d) as the templates, and the oligonucleotide complementary to the synthetic RNA used in step (c) or to a portion thereof, and an oligo-d(T) adapter as the primers;
(2) the method of (1) ; wherein the alkaline phosphatase used in step (a) is bacterial alkaline phosphatase (BAP);
(3) the method of (1) or (2), wherein the acid pyrophosphatase used in step (b) is tobacco acid pyrophosphatase (TAP);
(4) the method of any one of (1) to (3), wherein the RNA sample of step (a) is total RNA;
(5) the method of any one of (1) to (4), wherein the acid pyrophosphatase treatment of step (b) is performed under a condition wherein the pH is higher than 6.0 and lower than 8.0;
(6) a cDNA library constructed by any one of the method of (1) to (5);
(7) a method for isolating a transcription regulatory region containing a promoter of a gene on the genome, wherein the method comprises the steps of:
   (a) determining the nucleotide sequence of a cDNA contained in the cDNA library of (6) ;
   (b) comparing the determined nucleotide sequence to a genomic DNA sequence corresponding thereto to identify the transcription initiation site on the genome; and,
   (c) isolating the genomic DNA fragment located upstream of the identified transcription initiation site.

In the method for constructing a cDNA library, at first, an RNA sample containing mRNA and other RNA is treated with alkaline phosphatase to remove phosphate groups of non-full-length mRNA molecules in the sample, in which the phosphate groups are present at the 5'-ends of such mRNA molecules (step (a)).

The insufficiency of the alkaline phosphatase reaction and degradation of mRNA after the alkaline phosphatase reaction is thought to cause a reduction in the fullness ratio of the oligo-capping cDNA library constructed according to conventional methods. The method of the present invention is characterized by performing the oligo-capping reaction using RNA samples containing RNA other than mRNA. According to the present method, the degradation of the mRNA in the reaction, which is a major cause of the reduction in fullness ratio in conventional methods, is remarkably suppressed. This prevention of mRNA (used as the template for cDNA) degradation is thought to be a relative prevention, because the RNA other that mRNA included in the RNA sample serve as competitive substrates for RNA degradation activity present in the enzyme preparations.

The term "RNA species other than mRNA" used for the method of the present invention include, for example, ribosomal RNA, RNA molecules without a poly (A) tail, and synthetic RNA (note that RNA molecules without poly (A) are, herein, included in the term "RNA species other that mRNA"). The RNA samples used in the present invention may be total RNA directly prepared from cells, or those prepared by mixing RNA sample preparation with other RNA.

The source of the alkaline phosphatase used in the method of the present invention is not limited, and includes, for example, alkaline phosphatase derived from bacteria (BAP), calf intestine, shrimps, and such. There is no particular limitation on the reaction conditions, so long as the dephosphorylation reaction is accomplished. For example, the reaction can be conducted under conditions described in the literature (Yutaka Suzuki and Sumio Sugano, (1996) "CDNA Cloning", pp. 46-51, YODOSHA).

According to the method of the present invention, following the treatment of step (a), the RNA sample is treated with acid pyrophosphatase to convert the CAP structures present at the 5'-ends of the full-length mRNAs in the sample into phosphate groups (step (b)).

The source of the acid pyrophosphatase used in the method of the present invention is not limited, and, for example, enzymes derived from tobacco (TAP) are preferably used. The acid pyrophosphatase reaction is preferably performed under conditions with a pH higher than 6.0 and lower than 8.0, which may drastically improve the efficiency of mRNA-decapping reaction compared to the conventional condition of pH 5.5.

Subsequently, in the method of the present invention, after the treatment of step (b), the RNA sample is treated with RNA Ligase to ligate synthetic oligo-RNA (oligo-capping linkers) to the 5'-ends of the mRNAs in the sample, in which the CAP structures at the 5'-ends of the mRNAs are converted into hydroxyl groups (step (c)).

For example , T4 phage enzyme are preferably used as the RNA Ligase in the method. To prevent contamination of RNase, for example the enzyme can be prepared from recombinants in which the gene of the T4 phage enzyme is introduced into an RNase-deficient *E*. *coli* strain (A19). There is no particular limitation on the conditions for the ligase reaction, so long as it allows ligation of the 3'-end hydroxyl group of RNA to a 5'-end phosphate group of another RNA. For example, the reaction can be performed under conditions described in the literature (Yutaka Suzuki and Sumio Sugano (1996) "cDNA Cloning", pp. 46-51, YODOSHA) . There is noparticular limitation on the synthetic oligo-RNA used for the ligase reaction.

Subsequently, according to the present method, poly (A) RNA is selected from the RNA sample following the treatment in step (c) (step (d)). Selection of poly (A) RNA is preferably carried out using standard techniques for poly (A) RNA selection, for example, methods such as those using oligo-dT cellulose column or oligo-dT latex.

Subsequently, reverse transcription is performed using the poly (A) RNA selected in step (d) as a template, and oligonucleotide complementary to the synthetic RNA used in step (c) or to a portion thereof and an oligo-d(T) adapter as the primers in the method of the present invention (step (e)).

The reaction condition for reverse transcription may vary depending on factors, such as the enzyme to be selected. For example, the reaction can be performed under a conditions described in the literature by Yutaka Suzuki and Sumio Sugano, (1996) "cDNA Cloning", pp. 46-51, YODOSHA.

The method of the present invention allows construction of a cDNA library with an extreme high fullness ratio at their 5'-ends. This enables one to easily identify the transcription initiation site, and the identification of the transcription initiation site allows cloning of an upstream region of the transcription initiation site (i.e. transcription regulatory region including the promoter) on the chromosomal DNA for each corresponding mRNA. Fragments of the transcription regulatory region containing the promoter region on the genome, as well as information thereof, can be obtained experimentally or by computer analysis from the full-length cDNA clones and sequence information of the 5'-end of the full-length cDNA clones.

Accordingly, the present invention also provides methods for isolating gene expression regulating regions on the genome by using cDNA prepared by the method of the present invention for constructing cDNA libraries. This method can be carried out by a method comprising the steps of: (a) determining the nucleotide sequence of a cDNA contained in the cDNA library constructed by the method of the present invention; (b) comparing the determined nucleotide sequence to the corresponding genomic DNA sequence to identify the transcription initiation site on the genome; and (c) isolating the genomic DNA fragment located upstream of the identified transcription initiation site.

"Isolation of transcription regulatory regions" and "identification and purification of transcription regulating factors" are described in "Current Protocols for Cellular Engineering, Cell Technology, Supplement 8, pp. 352-398, Department of Oncology, The Institute of Medical Science, The University of Tokyo (ed.) (1993) SHUJUNSHA". The method described in the reference utilizes the S1 mapping method and the like for the identification of transcription initiation sites. On the other hand, the utilization of the method of the present invention will significantly facilitate the identification of transcription initiation sites, since cDNA libraries with extremely high fullness ratio at the 5'-ends can be constructed.

To obtain accurate information about genes expressed in a site-specific manner, analysis of such genes expressed in regions as defined as possible is required. However, since full-length cDNA libraries cannot be constructed by conventional methods unless using a large amount of samples, it is difficult to obtain accurate information about site-specific expression due to contamination of information about genes expressed in surrounding sites. By virtue of the method of the present invention, cDNA libraries having a high fullness ratio can be constructed, even from a small amount of biological sample. Thus, accurate information on transcription initiation sites of the genes expressed in a site-specific manner can be obtained based on the full-length cDNA library constructed from a more defined tissue section compared to conventional methods according to the present invention.

Current information on the relation between genomic DNA and cDNA, methodologies and computational analyses are given in "Genome Analysis: A Laboratory Manual volume 1-4" (Cold Spring Harbor Laboratory Press, volume 1 (1997), volume 2 (1998), and volumes 3-4 (1999))" and "Methods in Enzymology Vol. 303, Section II. Gene Identification" (Academic Press (1999), p77-161). These methods enable cloning of the regions upstream of the transcription initiation sites located at the 5'-ends of the cDNAs.

In addition, Human Genome Project has been in progress according to a plan to accomplish the whole genome sequencing by first mapping the BAC (bacterial artificial clone) clones into which pieces of human genome are cloned on the chromosome, and then, analyzing the sequence of each BAC clones mapped on the chromosome. Therefore, determination of the 5'-end sequence of the mRNA enables identification of transcription initiation sites on the human chromosome using the BAC clone, in which a human chromosomes about 150 kb in length has been cloned.

The method of the present invention can be, for example, carried out as follows. A cDNA library is constructed from any organ or cells according to the method of the present invention. The 5'-end sequence of each cDNA clone is determined by single-pass sequencing using a DNA sequencer (usually, several hundred nucleotides can be determined). Then, the transcription regulatory regions are isolated by whether the *in silico* searching method or an experimental methods for cloning transcription regulatory regions. More specifically, according to the former method, sequences identical to the 5'-end sequence of each cDNA clone are searched from genomic sequences and such in the public databases using BLAST algorithm (S. F. Altschul et al., J. Mol. Biol. 215:403-410 (1990)). When identical sequences are identified, the sequence of the transcription regulatory region is gained by obtaining the upstream region of the identical sequence for some kbs (typically, a length of 1 to 2 kb is sufficient). The latter methods include such method as cloning from BAC clones into which pieces of genomic DNA are cloned using the 5'-end sequence of each cDNA as a probe.

A much easier method than above can be mentioned as follows. J. M. Valdes et al. (Proc. Natl. Acad. Sci. USA, 91:5377-5381 (1994)) have developed a method for cloning genomic DNA flanking a CpG island existing before or after the transcription initiation site from YAC (yeast artificial chromosome) or cosmid clones into which pieces of human genome are cloned. More specific transcription regulatory regions may be obtained by using the 5'-end sequence of cDNA instead of a CpG island sequence. Templates other than YACs or cosmids can be used in the method. BAC clones can be-also used as the template.

A random-adapter primer described by Y. Suzuki et al. (Gene, 200: 149-156 (1997)) can be used as the 3'-end primer of mRNA in place of the oligo-dT primer in the cDNA library construction of the present invention for cloning a transcription regulatory region.

### Brief Description of the Drawings

Figure 1 is a schematic diagram depicting the principle of the oligo-capping method.
Figure 2 is a photograph comparing RNA ligation reactions using poly (A) RNA and total RNA as substrates. Samples applied to respective lanes were treated as follows:
   - lane 1: poly (A). RNA purified from 10 µg of cytoplasmic total RNA was ligated to 24 pmol of DIG-labeled oligo-RNA in a reaction system of 25 µl, and then was subjected to electrophoresis;
   - lane 2: 10 µg of cytoplasmic total RNA was ligated to 24 pmol of DIG-labeled oligo-RNA in a reaction system of 200 µl, and then was subjected to electrophoresis; and
   - lane 3: 10 µg of cytoplasmic total RNA was ligated to 24 pmol of DIG-labeled oligo-RNA in a reaction system of 300 µl, and then was subjected to electrophoresis.

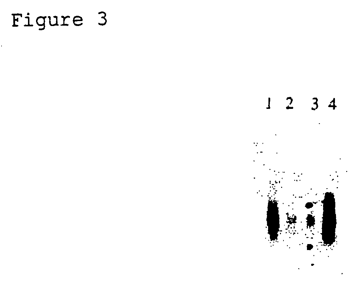

Figure 3 is a photograph comparing the result of ligation to DIG labeled oligo-RNA of cytoplasmic total RNAs after dephosphorylation with different amounts of BAP enzyme. Samples applied to respective lanes were treated as follows:
   - lane 1: 100 µg of cytoplasmic total RNA was reacted with 3.75-U of BAP in a reaction system of 150 µl for one hour at 37°C, followed by ligation to DIG-labeled oligo-RNA in a reaction system of 300 µl. One-tenth of the sample was applied to the gel;
   - lane 2: 100 µg of cytoplasmic total RNA was reacted with 5.0 U of BAP in a reaction system of 200 µl for one hour at 37°C, followed by ligation to DIG-labeled oligo-RNA in a reaction system of 300 µl. One-tenth of the sample was applied to the gel;
   - lane 3: 100 µg of cytoplasmic total RNA was reacted with 6.25 U of BAP in a reaction system of 250 µl for one hour at 37°C, followed by ligation to DIG-labeled oligo-RNA in a reaction system of 300 µl. One-tenth of the sample was applied to the gel; and,
   - lane 4: 100 µg of cytoplasmic total RNA was ligated to DIG-labeled oligo-RNA in a reaction system of 300 µl without the treatment with BAP. One-tenth of the sample was applied to the gel.

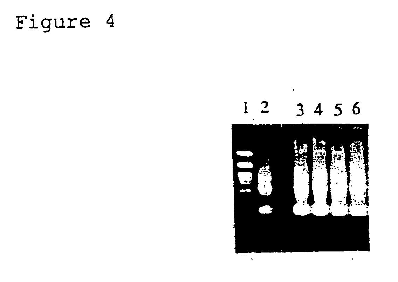

Figure 4 is a photograph comparing the state of RNA preparations of cytoplasmic total RNA dephosphorylated with different amounts of BAP enzyme. The samples applied to respective lanes were as follows:
   - lane 1: Molecular weight marker;
   - lane 2: 20 µg of cytoplasmic RNA was subjected to electrophoresis;
   - lane 3: 100 µg of cytoplasmic total RNA was reacted with 3.75 U of BAP in a reaction system of 150 µl for one hour at 37°C. Two-fifth volume of the sample was subjected to agarose gel electrophoresis; lane 4: 100 µg of cytoplasmic total RNA was reacted with 5.0 U of BAP in a reaction system of 200 µl for one hour at 37°C. Two-fifths of the volume of the sample was subjected to agarose gel electrophoresis;
   - lane 5: 100 µg of cytoplasmic total RNA was reacted with 6.25 U of BAP in a reaction system of 250 µl for one hour at 37°C. Two-fifths of the volume of the sample was subjected to agarose gel electrophoresis; and,
   - lane 6: 100 µg of cytoplasmic total RNA was incubated in the BAP buffer for one hour at 37°C. Two-fifths of the volume of the sample was subjected to agarose gel electrophoresis.

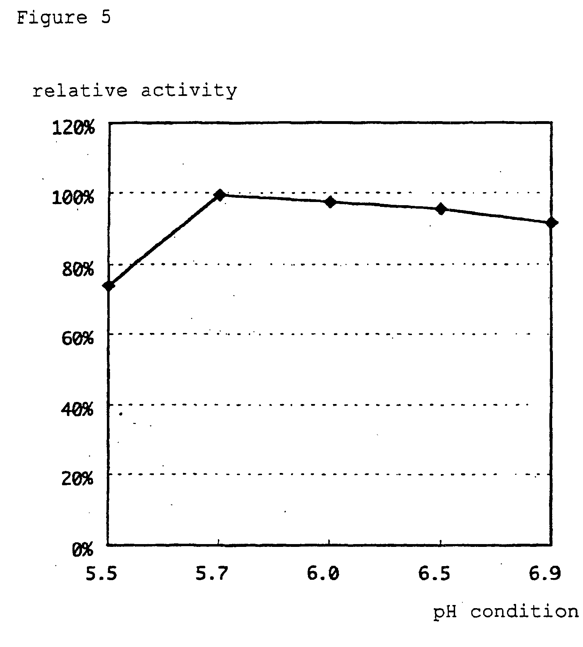

Figure 5 is a graph showing the pH-dependence of TAP enzyme activity of fraction A using 1.5 mM pNT as substrate. The values are indicated by relative activity taking the highest enzyme activity as 100%.

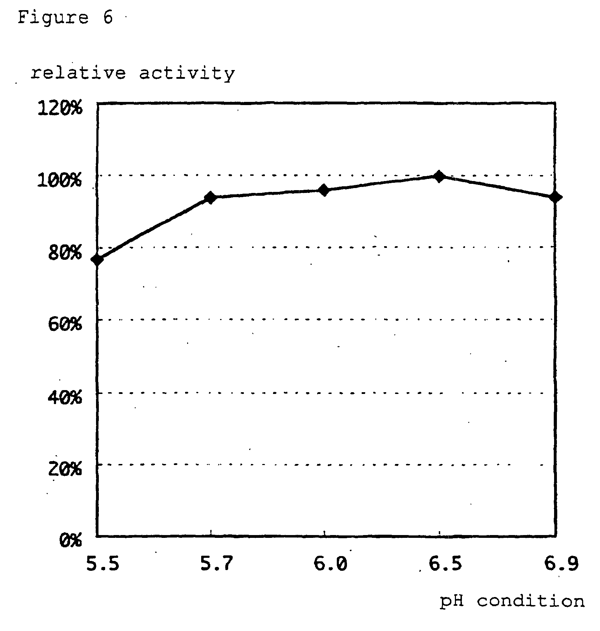

Figure 6 is a graph showing the pH-dependence of TAP enzyme activity of fraction B using 1.5 mM pNT as substrate. The values are indicated by relative activity taking the highest enzyme activity as 100%.

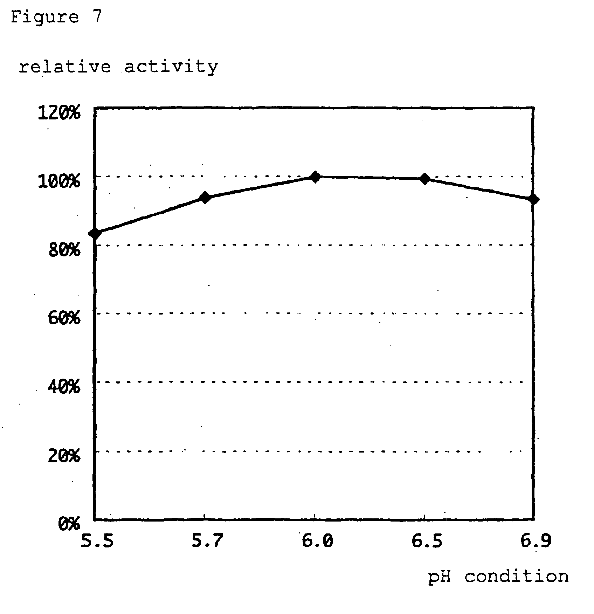

Figure 7 is a graph showing the pH-dependence of TAP enzyme activity of fraction C using 1.5 mM pNT as substrate. The values are indicated by relative activity taking the highest enzyme activity as 100%.

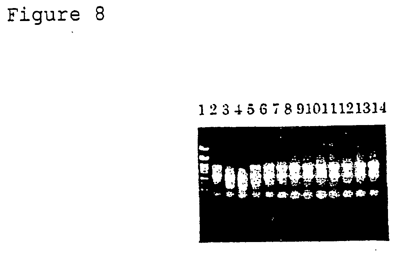

Figure 8 is a photograph showing the pH-dependence of RNA-degrading activity of the TAP activity fraction B. Samples applied to respective lanes were as follows:
   - lane 1: Molecular weight marker;
   - lane 2: 20 µg of cytoplasmic RNA;
   - lane 3: 2.0 U of fraction B enzyme and TAP buffer adjusted to pH 5.5 were added to 20 µg of cytoplasmic RNA, and was incubated at 37°C in for 16 hours;
   - lane 4: TAP buffer adjusted to pH 5.5 was added to 20 µg of cytoplasmic RNA, and was incubated at 37°C for 16 hours;
   - lane 5: 2.0 U of fraction B enzyme and TAP buffer adjusted to pH 5.7 were added to 20 µg of cytoplasmic RNA, and was incubated at 37°C for 16 hours;
   - lane 6 : TAP buffer adjusted to pH 5. 7 was added to 20 µg of cytoplasmic RNA, and was incubated at 37°C for 16 hours;
   - lane 7: 2.0 U of fraction B enzyme and TAP buffer adjusted to pH 6.1 were added to 20 µg of cytoplasmic total RNA, and was incubated at 37°C for 16 hours;
   - lane 8: TAP buffer adjusted to pH 6 .1 was added to 20 µg of cytoplasmic RNA, and was incubated at 37°C for 16 hours;
   - lane 9: 2.0 U of fraction B enzyme and TAP buffer adjusted to pH 6.5 were added to 20 µg of cytoplasmic RNA, and was incubated at 37°C for 16 hours;
   - lane 10: TAP buffer adjusted to pH 6.5 was added to 20 µg of cytoplasmic RNA, and was incubated at 37°C for 16 hours;
   - lane 11: 2.0 U of fraction B enzyme and TAP buffer adjusted to pH 6.8 were added to 20 µg of cytoplasmic RNA, and was incubated at 37°C for 16 hours;
   - lane 12 : TAP buffer adjusted to pH 6. 8 was added to 20 µg of cytoplasmic RNA, and was incubated at 37°C for 16 hours;
   - lane 13: 2.0 U of fraction B enzyme was added to 20 µg of cytoplasmic RNA, and was incubated at 37°C for 16 hours; and
   - lane 14: 20 µg of cytoplasmic RNA alone was incubated at 37°C for 16 hours.

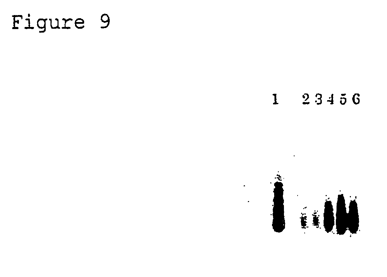

Figure 9 is a photograph comparing the result of ligation to the DIG-labeled oligo RNA of total cytoplasmic RNA samples that were decapped by TAP enzyme under different pH after the BAP treatment. Samples applied to respective lanes were as follows:
   - lane 1: Molecular weight marker;
   - lane 2: 100 µg of total cytoplasmic RNA treated with BAP was decapped with fraction B enzyme in a TAP buffer adjusted to pH 5.5, and then, was ligated to DIG-labeled oligo-RNA;
   - lane 3: 100 µg of total cytoplasmic RNA treated with BAP was decapped with fraction B enzyme in a TAP buffer adjusted to pH 5.7, and then, was ligated to DIG-labeled oligo-RNA;
   - lane 4: 100 µg of total cytoplasmic RNA treated with BAP was decapped with fraction B enzyme in a TAP buffer adjusted to pH 6.0, and then, was ligated to DIG-labeled oligo-RNA;
   - lane 5: 100 µg of total cytoplasmic RNA treated with BAP was decapped with fraction B enzyme in a TAP buffer adjusted to pH 6.5, and then, was ligated to DIG-labeled oligo-RNA; and
   - lane 6: 100 µg of total cytoplasmic RNA treated with BAP was decapped with fraction B enzyme in a TAP buffer adjusted to pH 6.8, and then, was ligated to DIG-labeled oligo-RNA.

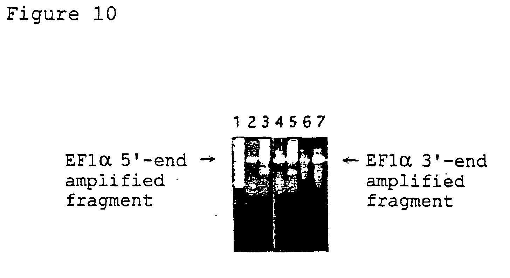

Figure 10 is a photograph showing the result of oligo-capping reactions using small amounts of RNA samples. 5 to 100 µg of cytoplasmic total RNA treated with BAP was decapped using fraction B enzyme in a TAP buffer adjusted to pH 6.5, and then, was ligated to oligo-RNA. The first strand cDNA was synthesized using the resultant RNA as the template and an oligo-dT primeras the primer. Using this first strand cDNA as the template, the 5'- and 3'-terminal regions of EF1α were amplified by PCR with combinations of primers FL3-666 (SEQ ID NO: 3) and EF1-1R (SEQ ID NO: 4), and primers EF1-3F (SEQ ID NO: 5) and FL3-705 (SEQ ID NO: 6), respectively, and then, agarose gel electrophoresis was conducted. Samples applied to respective lanes are described below. The positions of amplified fragments of EF1α, corresponding to 650 nucleotides at the 5'-end and 644 nucleotides at the 3'-end, are respectively indicated by arrows.
   - lane 1: Molecular weight marker;
   - lane 2: The 5'-terminal region of EF1α amplified in the oligo-capped library constructed from 100 µg of total cytoplasmic RNA;
   - lane 3: The 3'-terminal region of EF1α amplified in the oligo-capped library constructed from 100 µg of total cytoplasmic RNA;
   - lane 4: The 5'-terminal region of EF1α amplified in the oligo-capped library constructed from 20 µg of total cytoplasmic RNA;
   - lane 5: The 3'-terminal region of EF1α amplified in the oligo-capped library constructed from 20 µg of total cytoplasmic RNA;
   - lane 6: The 5'- terminal region of EF1α amplified in the oligo-capped library constructed from 5 µg of total cytoplasmic RNA; and
   - lane 7: The 3'-terminal region of EF1α amplified in the oligo-capped library constructed from 5 µg of total cytoplasmic RNA.

### Best Mode for Carrying out the Invention

The present invention will be described below in detail with reference to examples, but is not limited thereto.

### Example 1: Evaluation of oligo-capping reaction using synthetic oligo-RNA labeled with digoxigenin (DIG)

To exactly estimate the fullness ratio of a cDNA library, sequences of a measure of clones from the library should be analyzed. Accordingly, it is quite inefficient to optimize the reaction conditions for each step of the multiple-step reaction, while giving feedback from the sequencing results. Thus, a system that visualizes the products of oligo-capping reaction with high sensitivity using synthetic oligo-RNA labeled with digoxigenin (DIG) has been developed in the present invention.

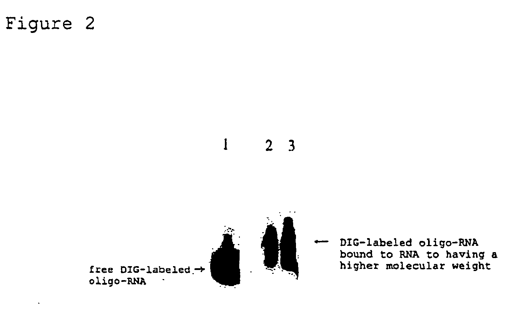

The 5'-end of synthetic oligo-RNA KMO2 (5'- AGC AUC GAG UCG GCC UUG UUG GCC UAC UGG -3'/ SEQ ID NO: 1) was labeled with DIG (Boehringer Mannheim). Oligo-capping reaction was carried out using this DIG-labeled synthetic oligo-RNA. 24 pmol DIG-labeled synthetic oligo-RNA was used for RNA corresponding to 0.2 µg of poly (A) RNA (approximately 10 µg of total RNA) . Following the RNA ligase reaction, the reaction product was loaded onto oligo-dT cellulose column type II (Collaborative Research) to purify poly (A) RNA. All of the poly (A) RNA was denatured by 18%(v/v) formaldehyde and electrophoresed on 1.0% (v/v) agarose in MOPS buffer (20 mM 2-morpholinopropanesulfonic acid (MOPS) , pH 7.0, 5 mM sodium acetate, 1 mM EDTA). Subsequently, the sample was blotted onto a positively charged nylon membrane (Boehringer Mannheim) by the capillary transfer method using 20x SSC buffer (3.6 M sodium chloride, 0.3 M sodium citrate, pH 7.0), followed by UV-cross-linking with Stratagene UV Cross-linker. The oligo-capping reaction products, RNA molecules bound by DIG-labeled synthetic oligo-RNA and unreacted synthetic oligo-RNA molecules, were detected on thus prepared filter using the DIG luminescence detection kit (Boehringer Mannheim). As shown in Figure 2, oligo-capped reaction products with a high molecular weight can be detected by performing synthetic oligo-RNA ligation using DIG labeled synthetic oligo-RNA.

As a result, the progress and the conditions of RNA of oligo-capping reaction at each stage are relatively easily estimated.

### Example 2: Optimization of RNA ligase reaction

Ligation of DIG-labeled synthetic oligo-RNA to the 5 '-phosphate ends of RNA molecules by RNA Ligase was carried out by using poly (A) RNA and total cytoplasmic RNA as the substrate. The conditions for the RNA ligase reaction were set according to the literature (Yutaka Suzuki and Sumio Sugano, (1996) "cDNA Cloning", pp. 46-51, YODOSHA). Poly (A) RNA purified from 10 µg of total cytoplasmic RNA using oligo-dT cellulose column type II (Collaborative Research) was used as the starting material, and was ligated to 24 pmol of DIG-labeled oligo-capping linker. Alternatively, 10 µg total cytoplasmic RNA was used as the starting material, ligated to 24 pmol DIG-labeled oligo-capping linker, and subsequently, poly (A) RNA was purified by poly (A) selection using oligo-dT cellulose column type II. As shown in Figure 2, oligo-capping reaction products with high molecular weight can be detected by performing ligation of oligo-capping linker using DIG-labeled synthetic oligo-RNA. It was revealed that efficient ligation of synthetic oligo-RNA to the 5'-phosphate ends is possible even when the total RNA is used for the reaction. Furthermore, when poly (A) RNA was used as the substrate, molecular weights of the labeled reaction products were significantly lower than those produced using total RNA as the substrate. According to the result, it was revealed that RNA degradation also occurs during the process of RNA ligase reaction, and that mRNA molecule degradation during the RNA ligase reaction is suppressed by using total RNA rather than poly (A) RNA.

### Example 3: Optimization of the conditions for BAP reaction

BAP treatment of total cytoplasmic RNA as the substrate was carried out, and then, DIG-labeled synthetic oligo-RNA was ligated by RNA Ligase. The reaction conditions for RNA ligase reaction were set according to the literature (Yutaka Suzuki and Sumio Sugano, (1996) "cDNA Cloning", pp. 46-51, YODOSHA). After ligating the DIG-labeled synthetic oligo-RNA, poly (A) selection was carried out to purify poly (A) RNA.

As shown in Figure 3, dephosphorylation of the 5'-phosphate ends of RNA occurred also when cytoplasmic total RNA was used as the substrate, and the DIG-labeled oligo-capped reaction products detected in high molecular weight fractions decreased. Moreover, molecular weight reduction assumed to result from RNA degradation during BAP reaction could not be observed when total cytoplasmic RNA was used as the substrate (Figure 4).

### Example 4: Purification of TAP from tobacco cell culture

It was suggested that the major cause of the decrease in fullness ratio of the library is the severe RNA degradation that occurs during the decapping reaction by TAP in the process of oligo-capping reaction. Thus, the inventors examined a variety of commercially available TAP preparations to compare their activity on RNA degradation, but failed to find a TAP preparation with efficiently low RNA degradation activity. Accordingly, the inventors cultured tobacco BY2 cells to purify TAP enzyme with low RNA-degradation activity.

Purification of TAP was carried out according to the method of Shinshi et al. (H. Shinshi et al. (1976) Biochemistry 15: 2185-2190) with partial modification. Tobacco BY2 cells were cultured using MS medium (Sigma) with shaking for 10 days at 28°C in the dark. Obtained cells were homogenized; precipitated with ammonium sulfate; and were purified using DEAE-Cellulose SH column (Wako Pure Chemical Industries), Phospho-Cellulose Pll column (Whattman), CM-Sepharose SH column (Pharmacia), and Sephadex G200 column (Pharmacia). TAP activity was detected in two active fractions, high and low molecular weight fraction distinctly separated by Sephadex G200 column. The low molecular weight fraction was further separated into two active fractions by DEAE-Cellulose SH column. Each fraction showed a decapping activity, and almost no difference in the decapping activity was observed among these fractions.

TAP activity was determined as follows : 1) reaction was conducted using 1.5 mM thymidine-5'-monophosphate p-nitroester (pNT, SIGMA) as the substrate at 30°C; 2) sodium hydroxide was added to a final concentration of 66 mM to stop the reaction; and 3) the amount of generated p-nitrophenol was determined by measuring the absorbance at 400 nm. The amount of the enzyme that can generate 1 µmol of p-nitrophenol from thymidine-5'-monophosphate p-nitroester in one minute is taken as 1 U of the enzyme activity. Calculation was made taking molecular extinction coefficient of p-nitrophenol as 18,000.

### Example 5: Optimization of TAP reaction

Shinshi et al. reports that the optimal pH for TAP reaction using NAD+ or pNT as the substrate is pH 5.3 and pH 6.0, respectively (H. Shinshi et al. (1976) Biochemistry 15: 2185-2190) . The pH conditions indicated in most protocols provided by the suppliers of commercially available TAP enzymes ranges from 5.5 to 6.0. Additionally, the protocol of the oligo-capping method reported by Sugano et al. above also indicates to perform TAP reaction at pH 5.5 (K. Maruyama and S. Sugano. (1994) Gene 138:171-174; Y. Suzuki et al. (1997) Gene 200:149-156; Kazuo Maruyama and Sumio Sugano "Oligo-capping and construction of full-length cDNA libraries using PCR", and Kazuo Maruyama and Sumio Sugano "Cloning of the 5 '- end of mRNA by replacement by an oligo-cap" (1996) Experimental Medicine Vol. 11, No. 18, pp. 2491-2495; Yutaka Suzuki and Sumio Sugano, (1996) "cDNA Cloning", pp. 46-51, YODOSHA; S. Kato et al. (1994) Gene 150:243-250; Attached instruction for Tobacco acid phosphatase (Wako Pure Chemical Industries); and Attached instruction for Tobacco acid phosphatase (Epicentre)). Investigation on the influence of reaction pH to the TAP activity of the active fractions purified in this experiment using pNT as the substrate indicated that all the fractions A, B, and C exhibit increased TAP activity at pH 5.5-6.9. When pNT was used as the substrate, the optimal pH was pH 5.7 for fraction A (Figure 5), pH 6.5 for fraction B (Figure 6), and pH 6.0 for fraction C (Figure 7).

Then, the effect of pH on RNA degradation during the process of TAP reaction was investigated. TAP enzyme of fraction B and TAP buffer adjusted to pH 5.5-pH 6.9 were added to 20 µg of cytoplasmic RNA, the mixture was incubated for 16 hours at 37°C, and degradation of RNA during the incubation was monitored. As a result, degradation of RNA was observed at a pH of 6.0 or less (Figure 8). Alternatively, TAP enzymes of fractions A and B were used to investigate the influence of pH on decapping reaction using RNA as the substrate. BAP treatment was carried out using total cytoplasmic RNA as the substrate, the reaction product was dissolved in a sodium acetate buffer adjusted between pH 5.5 and 6.9, and then, was reacted with TAP for one hour at 37°C. Following the TAP reaction, the reaction products were ligated to DIG-labeled synthetic oligo-RNA by RNA Ligase, and then were analyzed. Investigation of purified reaction product labeled with DIG revealed that mRNA was decapped most efficiently when TAP treatment was performed at pH 6.5 (Figure 9). Further investigation using densitometry indicated that decapping activity at pH 6.5 was higher more that three-fold than the activity at the conventional condition of pH 5.5. From the results described above, decapping reaction at pH 6.5 is anticipated to improve both the fullness ratio and clone titer of the constructed library.

### Example 6: Evaluation of oligo-capping reaction by the modified protocol

The oligo-capping reaction was carried out using 5 to 100 µg of total cytoplasmic RNA as the starting material. The first strand cDNA was synthesized using the reaction product as the template and oligo-dT adaptor-primer FL-706L (5'-GCG GCT GAA GAC GGC CTA TGT GGC CTT TTT TTT TTT TTT TTT -3'/SEQ ID NO: 2) as a primer. Oligo-capped mRNA samples were dissolved in the first-strand cDNA synthesis buffer (Life Technologies; 50 mM Tris-HCl buffer, pH 8.3, 75 mM potassium chloride, 3 mM magnesium chloride, 0.8 mM dNTP, 12 mM dithiothreitol, 0.5 µl RNase inhibitor) to a final volume of 25 µl, were incubated for one hour at 16 °C with the addition of 5 pmol oligo-dT adaptor-primer FL-706L and 200 U Superscript II (Life Technologies), and then, were reacted for an hour at 42°C. The mixture was treated with phenol-chloroform following the addition of 25 µl H₂O. After the phenol-chloroform treatment, the mixture was incubated for 60 minutes at 65°C with the addition of 7.5 µl 0.1 N NaOH and 1 µl 0.5 M EDTA, thereby degrading RNA chains. The resultant solution was treated with phenol-chloroform, precipitated with ethanol, and then, was dissolved in 50 µl TE buffer (pH 8.0). Other reaction conditions were set according to the literature (Yutaka Suzuki and Sumio Sugano, (1996) "cDNA Cloning", pp. 46-51, YODOSHA).

PCR was performed using thus generated first strand cDNA as the template and a combination of oligo-capping linker-primer FL3-666 (5'- AGC ATC GAG TCG GCC TTG TTG -3'/SEQ ID NO: 3) and EF1α primer EF1-1R (5'-TGC TAC TGT GTC GGG GTT GTA-3'/SEQ ID NO: 4) or EF1 α primer EF1-3F (5'-CCT GAA CCA TCC AGG CCA AAT-3'/SEQ ID NO: 5) and oligo-dT adaptor-primer FL3-705 (5'-GCG GCT GAA GAC GGC CTA TGT -3'/SEQ ID NO: 6). As a result of PCR, a fragment of 650 nucleotides at the 5'-end or a fragment of 644 nucleotides at the 3'-end of the EF1α gene were amplified. One fortieth of the first strand cDNA (1.25 µl) was dissolved in PCR buffer (10 mM Tris-HCl buffer, pH 8.3, 50 mM potassium chloride, 1. 5 mM magnesium chloride, and 0.2 mM dNTP) to give a reaction volume of 25 µl, and the reaction was carried out by adding 2.5 pmol of each primer and 2.5 U of TaKaRa Taq (TaKaRa). The reaction was conducted using Perkin-Elmer Model 9600, and after incubation for 5 minutes at 95°C, 17 or 22 cycles of reaction at 95°C for 30 seconds, 52°C for one minute, and 72°C for one minute was conducted. After the PCR, one-fifth of the reaction product (5 µl) was subjected to electrophoresis on 2.0%(w/w) agarose gel using TBE buffer (45 mM Tris-borate buffer, 2 mM EDTA pH 8.0) to verify the amplification of each fragment. Other reaction conditions were set according to the literature (Yutaka Suzuki and Sumio Sugano, (1996) "cDNA Cloning", pp. 46-51, YODOSHA).

The ratio of amplified fragments at the 5'-end and those at the 3'-end was almost 1:1, when 20 to 100 µg RNA was used in the PCR with 17 cycles (Figure 10), suggesting that nearly 100% of the full-length mRNAs were oligo-capped at their 5'-ends. Furthermore, PCR with 17 cycles using 5 µg of cytoplasmic total RNA also allowed clear detection of the amplified fragments from the first strand cDNA corresponding to EF1α mRNA to which the oligo-cap linker had been added to the 5'-end.

### Example 7: Construction of oligo-capped cDNA library

NT2, SK-N-MC, and H4 cells were cultured to extract the total cytoplasmic RNA according to the method described by Maniatis (Sambrook, J. et al. "Molecular cloning, a laboratory manual/ second edition", Cold Spring Harbor Laboratory Press, 1989 Chapter 8.3.).

In addition, total RNA samples from human adrenal gland (64016-1), human whole brain (64020-1), human liver (64022-1), human spleen (64034-1), human thymus (64028-1), and human trachea (64091-1) were purchased from Clonetech. 100, 50, and 5 µg of total cytoplasmic RNA from NT2 cells, and 100 µg of total cytoplasmic RNA from the other cells were used in the experiment. Protocol for constructing oligo-capped cDNA libraries using 100 µg total cytoplasmic RNA as the starting material is described below. All the reaction volumes were reduced to 1/2 and 1/20, respectively, when 50 µg and 5 µg of total cytoplasmic RNA was used as the starting material.

Total cytoplasmic RNA (100 µg) was dissolved in BAP buffer (0.1 MTris-HCl buffer, pH 7 . 0 , 10 mM 2-mercaptoethanol , 216 U RNase inhibitor (Promega)) to give a reaction volume of 200 µl, and was reacted with 5 U of BAP (RNase-free, TaKaRa) for 60 minutes at 37°C. The mixture was treated with phenol-chloroform, precipitated with ethanol, and dissolved in TAP buffer (50 mM sodium acetate, pH 6.5, 1 mM EDTA, 10 mM 2-mercaptoethanol and 108 U RNase inhibitor' (Promega)) to a reaction volume of 100 µl. One unit of TAP enzyme purified from tobacco cells was added to the solution, and was reacted for 60 minutes at 37°C. The reaction was treated with phenol-chloroform, precipitated with ethanol. Subsequently, the precipitate was dissolved in RNA Ligase buffer (50 mM sodium acetate, pH 7.0, 10mM 2-mercaptoethanol, 5 mM MgCl₂, 0.5 mM ATP, 300 U RNase inhibitor (Promega) and 25% polyethylene glycol 8000); mixed with 120 pmol synthetic oligo-RNA KM-02 (5'- AGC AUC GAG UCG GCC UUG UUG GCC UAC UGG -3'/ SEQ ID NO: 1) and 525 U RNA Ligase (RNase-free, TaKaRa) ; and then, was incubated for three hours at 20°C. 600 µl of H₂O was added to this reaction, treated with phenol-chloroform, and precipitated with ethanol. Subsequently, the precipitate was dissolved in 2 ml column-loading buffer (20 mM Tris-HCl, pH 7.6, 0.1 M sodium chloride, 1 mM EDTA and 0.05% sodium N-sarcosinate), and was loaded onto,oligo-dT cellulose column type II (Collaborative Research) equilibrated with column-loading buffer. The column was washed with 4.5ml column loading buffer, eluted with 900 µl elution buffer (10 mM Tris-HCl, pH 7.6, 1 mM EDTA and 0.05% SDS), and oligo-capped poly (A) fractions were recovered.

Total volume of the oligo-capped mRNA thus obtained (approx. 1 µg) was suspended in the first-strand cDNA synthesis buffer (mixture of 50 mM Tris-HCl buffer, pH 8.3, 75 mM potassium chloride , 3 mM magnesium chloride, 12 mM dithiothreitol, and 0.8 mM dNTP) to give a reaction volume of 25 µl. 5 pmol of oligo-dT adaptor-primer FL3-705 (5'-GCG GCT GAA GAC GGC CTA TGT GGC CTT TTT TTT TTT TTT TTT -3'/ SEQ ID NO: 2) and 200 U of Superscript II were added to the suspension. The mixture was reacted for one hour at 16°C and subsequently for another hour at 42°C. Equal volume of H₂O was added to the reaction, and the mixture was treated with phenol-chloroform, followed by incubation with 7.5 µl of 0.1 N NaOH and 1 µl of 0.5 M EDTA for 60 minutes at 65°C. Then, 10 µl of 1 M Tris-HCI buffer (pH 7.8) was added, and subsequently 35 µl of 7.5 M ammonium acetate and 250 µl of ethanol were added for ethanol precipitation. The obtained resultant precipitate was dissolved in 50 µl of TE buffer (pH 8.0) . High-fidelitylong PCR was performed using 1/5 volume (10 µl) of the first strand cDNA as the template, and oligo-capping linker-primer FL3-666 (5'- AGC ATC GAG TCG GCC TTG TTG -3'/ SEQ ID NO: 3) and oligo-dT adaptor-primer FL3-705 (5'-GCG GCT GAA GAC GGC CTA TGT -3'/ SEQ ID NO: 6). PCR was performed using GeneAmp XL PCR kit (Perkin-Elmer) and 8 pmol of each primer was added to a system having a volume of 100 µl. The reaction was conducted using Perkin-Elmer Model 9600, after incubation for 5 minutes at 95°C, 15 cycles of a reaction at 95°C for one minute, 58°C for one minute and 72°C for ten minutes were conducted. Following the phenol-chloroform treatment and ethanol precipitation of the obtained reaction product, the whole reaction product was dissolved in NEB buffer 2 (New England Biolabs; 10 mM Tris-HCl buffer, pH 7.9, 10 mM magnesium chloride, 50 mM sodium chloride, 1 mM dithiothreitol, and 1 mg/ml (w/v) bovine serumalbumin) supplemented with 100 µl of bovine serum albumin. Forty units of SfiI (New England Biolabs) was added to the mixture, which was reacted for 3.5 hours at 50°C to cleave the SfiI site that were designed within the oligo-capping linker at the 5'-end and the oligo-dT adaptor at the 3'-end. After phenol-chloroform treatment and ethanol precipitation, 1 kb or longer fragments resolved by agarose gel electrophoresis were recovered with GeneClean (Bio 101). Half volume of the sample was mixed with about 50 ng cloning vector pME18SFL3 cleaved with DraIII, followed by a reaction using DNA Ligation Kit, version 1 (TaKaRa) for 15 hours at 16°C with a reaction volume of 30 µl.

### Example 8: Estimation of the fullness ratio of the constructed cDNA libraries

A part of the constructed cDNA libraries was used to transform *E*. *coli* strain DH10B by electroporation using BioRad E. coli Pulser, and transformants were obtained. About 800,000 to 3,300,000 clones of transformants could be obtained from the library constructed by 15-cycle PCR using 100 µg oligo-capped total RNA, calculated based on the RNA used (50 or 100 µg of total cytoplasmic RNA) (Table 1). These transformants were cultured in LB medium containing 50 µg/ml ampicillin with shaking at 37°C overnight, and plasmid DNA was extracted from them using the Kurabo automated plasmid isolation system, PI sigma 1000. The 5'-end sequences of the cDNA in these plasmid clones were determined by single-pass sequencing using the ABI Big Dye Terminator kit and the ABI PRISM 377 automated DNA sequencer. Homology search in the GenBank database for these sequences were conducted using Blast 2.0 released from. Washington University. The 5'-ends of the clones were analyzed to determine whether they contain the full-length sequence by comparing the 5'-end nucleotide sequence between the sequence in clones that were judged to contain the same gene corresponding to any known mRNA deposited in GenBank and those of known mRNA. That is, if the 5'-end nucleotide sequence of a clone is longer than the corresponding sequence in GenBank, such a clone was regarded as a "full-length clone at the 5'-end". When a sequence contained the translation initiation codon of the corresponding sequence in GenBank, such a sequence was regarded as a "full-length clone having the translation initiation site". When a sequence lacked the translation initiation codon, such a clone was regarded as an "non-full-length clone". For convenience, fullness ratio in the library (percentage of full-length clones) was calculated as (full-length clones having the translation initiation site)/(full-length clones having the translation initiation site + non-full-length clones).

The fullness ratio of the libraries, including the "full-length clones having the translation initiation site", constructed using 100 or 50 µg of total cytoplasmic RNA (representing about 2 and 1 µg of poly (A) RNA, respectively) was approximately 89% to 99% (Tables 1 and 2).

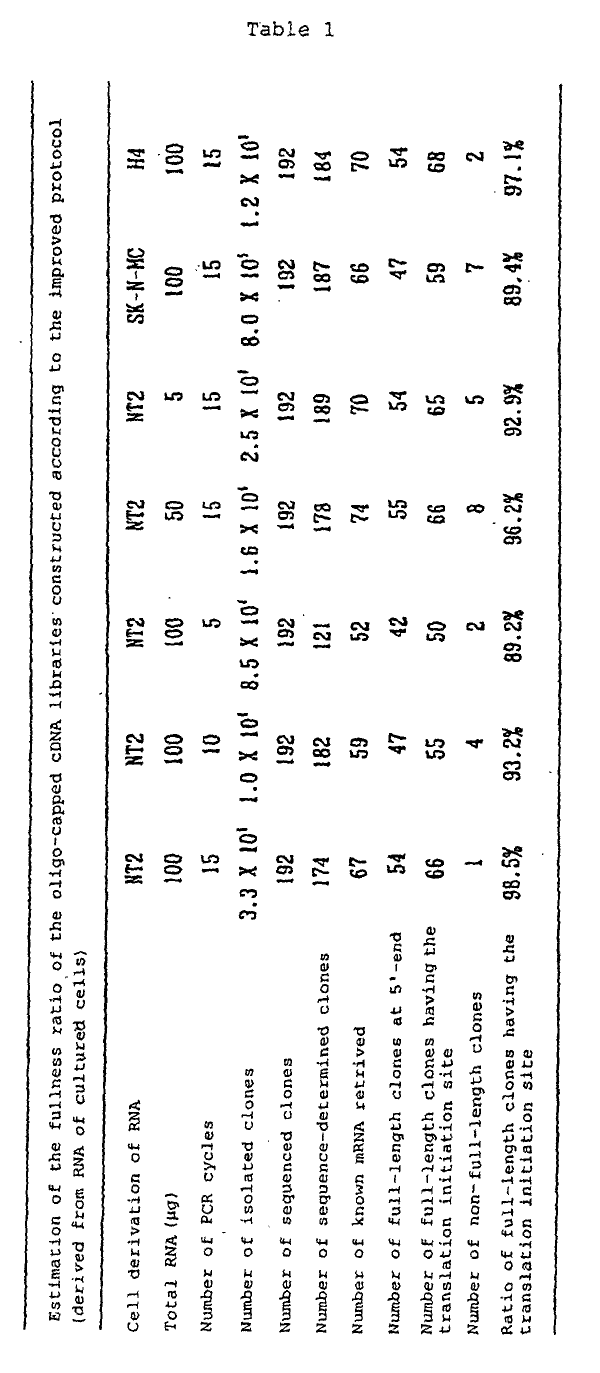

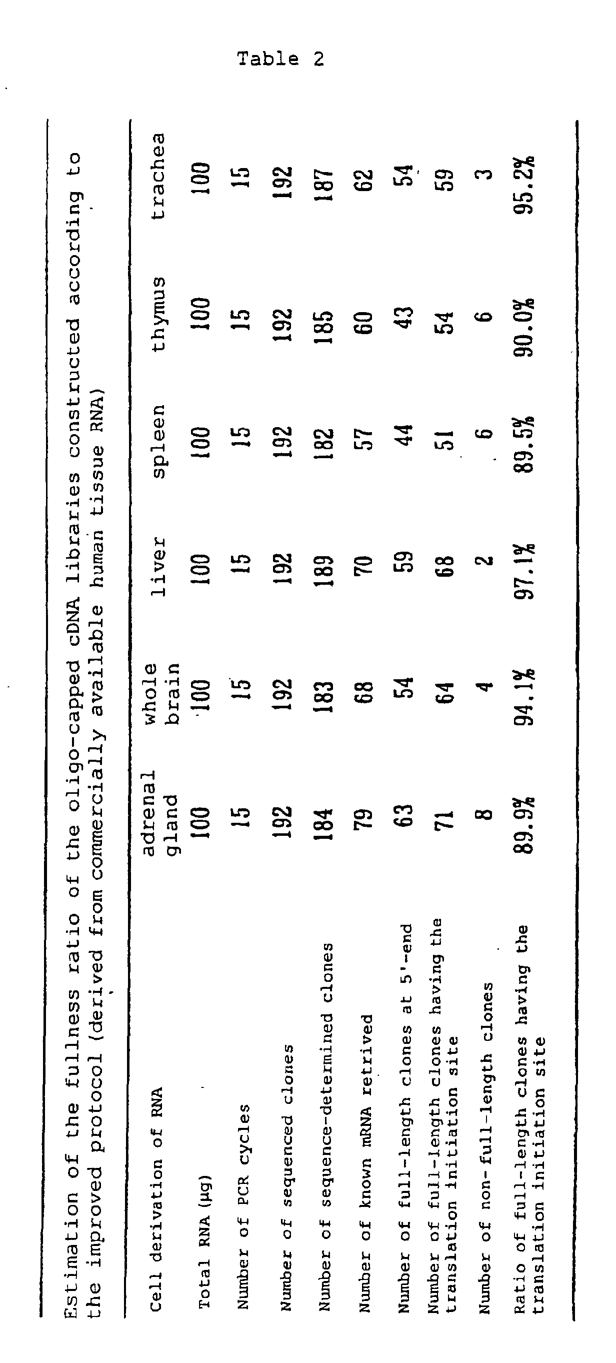

These values were remarkably higher than the results, 60 to 75%, obtained by analyzing the oligo-capped cDNA libraries constructed from a larger number of biological samples using conventional methods (T. Ota et al. (1998) Microbial and comparative genomics 3:C-87). According to the results described above, it was indicated that the fullness ratio of the clones obtained by the protocol improved in this study increased compared to those obtained by conventional protocols. Furthermore, it was revealed that cDNA libraries with high fullness ratio from smaller amount of RNA samples can be constructed according to the present method.

In addition, this study also revealed that commercially available RNA preparations, which have been considered unsuitable sources for constructing full-length cDNA libraries due to contamination of degraded RNA molecules, can be used for constructing full-length libraries with high-quality.

### Example 9: Number of the clone titer obtained by reduced PCR cycles for synthesis of second strand cDNA

100 µg of total RNA from NT2 cells were oligo-capped, and cDNA library was constructed by performing 10 and 5 cycles of PCR. A part of the cDNA library was electroporated into cells of *E*. *coli* strain DH10B by BioRad E. coli Pulser to obtain transformants. As a result, 1,000,000 and 85,000 clones of transformants could be obtained, respectively, calculated based on 100 µg of total RNA.

### Industrial Applicability

By virtue of the present invention, mRNA degradation during the reaction, which is a major cause of the reduction in the fullness ratio reached by the conventional methods, can be prominently suppressed. By comparing to the cDNA libraries constructed by conventional methods, the fullness ratio of the cDNA libraries constructed by the method of present invention were shown to be markedly increased. According to the analysis carried out by the inventors, the fullness ratio of the cDNA libraries constructed by the method of present invention were approximately 89 to 99%, even when the volumes of starting materials were reduced to one-fifth to one-hundredth of the volume used in conventional methods, while the fullness ratio of the oligo-capped cDNA libraries constructed by conventional methods were approximately 60 to 75%.

According to the method of the present invention, the efficiency of decapping reaction of mRNA having CAP structure has been tripled or more by modifying the pH conditions during the acid pyrophosphatase reaction, thereby dramatically increasing the clone titers obtained as full-length cDNA by the construction of the cDNA libraries. This enabled the construction of oligo-capped libraries with high clone titer from a smaller quantity of starting materials. According to the method of the present invention, a sufficient number of transformants (more than one million clones) can be obtained even when the libraries are constructed from a starting material with an RNA volume reduced to one-tenth of those used for conventional methods.

The fact that the polymerase chain reaction is performed during the process of cDNA construction is pointed out as one of the problems in the oligo-capping method. There are apprehensions about polymerase chain reaction, like that artificial mutations might be introduced during in vitro DNA replication, that bias may be caused by preferential amplification of particular fragments, especially small fragments, and the like. These problems are alleviated according to the method of the present invention by reducing the number of PCR cycles during construction of the cDNA library which was accomplished due to the increase of the oligo-capping efficiency by altering the conventional conditions for acid pyrophosphatase reaction. In fact, when a library was constructed from RNA of a volume that is 1/10 that used for conventional methods, approximately 85,000 clones were obtained, even if the number of PCR cycles was reduced to as few as five.

The method of the present invention for constructing full-length cDNA libraries makes it possible to construct a cDNA library from a small quantity of RNA, which was extremely difficult according to convention methods. This feature suggests that the present invention may serve as an extremely important basic technology in the fields of academic research, for elucidating the functions of a large number of genes emerged from genome analyses, for the purpose of efficient acquisition of full-length cDNA for industrially useful genes, and for development of kits for constructing full-length cDNA libraries in order to support these academic research and research activities for industrialization and commercial supply of high quality cDNA libraries as genetic resources.

Furthermore, a method for isolating transcription regulatory regions containing promoters on the genomes has been provided, which utilizes cDNA generated by the method of the present invention as described above. There is a plan which aims for completion of a rough draft of human genome (slightly inaccurate human genome sequence analysis) covering more than 90% of chromosomes till the spring of year 2000, and accomplishment of the entire human genome sequence analysis by the year 2003. It is considerably difficult to elucidate the transcription initiation sites with the aid of analysis softwares from human genome in which long stretches of introns are dispersed. However, the method of the present invention for constructing cDNA libraries will facilitate acquisition of genomic regions located in the upstream regions of translation initiation sites, which are involved in transcriptional control, because transcription initiation sites on the genome may be easily identified from the 5'-end sequences of full-length cDNAs.

## Claims

1. A method for constructing a cDNA library, comprising the steps of,
(a) treating the RNA sample containing mRNA and other RNA with alkaline phosphatase to remove phosphate groups from non-full-length mRNA molecules having phosphate groups at the 5'-ends,
(b) following the treatment of step (a), treating the RNA sample with acid pyrophosphatase to convert the CAP structures of the full-length mRNAs in the sample into phosphate groups, wherein the full-length mRNAs have a CAP structures at their 5'-ends,
(c) following the treatment of step (b), treating the RNA sample with RNA Ligase to ligate synthetic oligo-RNA (oligo-capping linkers) to the 5'-ends of mRNAs in the RNA sample, wherein the CAP structures of the mRNAs at the 5'-end are converted into phosphate groups,
(d) selecting poly (A) RNAs from the RNA sample following the treatment of step (c),
(e) performing reverse transcription using the poly (A) RNAs selected in step (d) as the templates, and the oligonucleotide complementary to the synthetic RNA used in step (c) or to a portion thereof, and an oligo-d(T) adapter as the primers.

2. The method of claim 1, wherein the alkaline phosphatase used in step (a) is bacterial alkaline phosphatase (BAP).

3. The method of claim 1 or claim 2, wherein the acid pyrophosphatase used in step (b) is tobacco acid pyrophosphatase (TAP).

4. The method of any one of claim 1 to claim 3, wherein the RNA sample of step (a) is total RNA.

5. The method of any one of claim 1 to claim 4, wherein the acid pyrophosphatase treatment of step (b) is performed under a condition wherein the pH is higher than 6.0 and lower than 8.0.

6. A cDNA library constructed by any one of the method of claim 1 to claim 5.

7. A method for isolating a transcription regulatory region containing a promoter of a gene on the genome, wherein the method comprises the steps of,
(a) determining the nucleotide sequence of a cDNA contained in the cDNA library of claim 6,
(b) comparing the determined nucleotide sequence to a genomic DNA sequence corresponding thereto to identify the transcription initiation site on the genome,
(c) isolating the genomic DNA fragment located upstream of the identified transcription initiation site.
